# EUROPÄISCHE PATENTSCHRIFT

(11) **EP 0 472 891 B1**
(45) Veröffentlichungstag und Bekanntmachung des Hinweises auf die Patenterteilung: **12.10.1994**
(21) Anmeldenummer: 91112096.2
(22) Anmeldetag: 19.07.1991
(51) Int. Cl.: G02B 23/26, A61B 1/00

(54) **Vorrichtung zur Verbindung einer Faseroptik mit einem Gerät, insbesondere einem medizinischen Instrument**
Arrangement for the connection of a fiber optic with an apparatus, particularly a medical instrument
Arrangement de connexion d'une fibre optique avec un appareil, en particulier un instrument médical

(30) Priorität: 21.08.1990 DE 4026451
(43) Veröffentlichungstag der Anmeldung: 04.03.1992
(73) Patentinhaber: Schott Glaswerke, 55122 Mainz (DE); Carl-Zeiss-Stiftung trading as SCHOTT GLASWERKE, 55122 Mainz (DE)
(72) Erfinder: Sternstein, Werner, W-6227 Hallgarten (DE)
(74) Vertreter: Fuchs Mehler Weiss

(56) Entgegenhaltungen:
- EP-A- 0 308 023
- DE-A- 3 405 803
- DE-A- 3 922 301
- US-A- 4 606 331
- PATENT ABSTRACTS OF JAPAN Band 13,Nr.326 (C-620),24. Juli 1989;JP-A--01104238

## Beschreibung

Die Erfindung betrifft eine Vorrichtung zur Verbindung einer Faseroptik mit einem Gerät, insbesondere einem medizinischen Instrument, nach dem Oberbegriff des Anspruches 1.

Bei bisherigen Vorrichtungen dieser Art handelt es sich entweder um Verbindungen, bei denen die Faseroptiken mit den Geräten, insbesondere den medizinischen Instrumenten, unlösbar vergossen oder verklebt sind. Ferner sind Klemmvorrichtungen oder Verschraubungen für Faseroptiken bekannt.

Die bisherigen Anordnungen haben jedoch den Nachteil, daß sie nach der Art der Verbindung und der Anschlüsse am Arbeitskanal des jeweiligen Gerätes beim Einführen abgedichtet werden müssen. Hieraus resultiert die Gefahr eines Bruches der Faseroptik, ungenügende bzw. fehlerhafte Abdichtung, die zu mangelnder Dichtheit führen kann sowie eine Endflächenverschmutzung der Faseroptik. Ferner sind diese Anordnungen unübersichtlich in der Handhabung und nicht universell einsetzbar.

Desweiteren ergibt sich der Nachteil, daß das distale Ende der Faseroptik in dem freien Lumen des jeweiligen Gerätes, insbesondere des medizinischen Instrumentes, nur schwer zu manipulieren ist, da die Positioniermöglichkeit äußerst schlecht ist.

Um die Spitze einer Glasfaser möglichst schadensfrei in ein an einem Gehäuse anzukoppelnden Rohr anzubringen, ist gemäß der DE 34 05 803 A1 vorgeschlagen worden, die Glasfaser mit einem Abstandshalter im Bereich des distalen Endes zu versehen.

Der Außendurchmesser dieses kugeligen oder zylindrischen Abstandshalters ist dabei so gewählt, daß er in dem Inneren des Rohres bewegbar ist. Durch Beaufschlagen des Abstandshaltersendes mit Preßgas von der proximalen Seite des Rohres her wird dieser zum distalen Ende des Rohres bewegt. Der Abstandshalter wirkt also quasi wie ein Kolben in einer Hülse.

Es ist die Aufgabe der vorliegenden Erfindung, eine Vorrichtung zur Verbindung einer Faseroptik mit einem Gerät, insbesondere einem medizinischen Instrument, der im Oberbegriff des Anspruches 1 angegebenen Art zu schaffen, die leicht zu handhaben ist und einen weiten Anwendungsbereich hat.

Die Lösung dieser Aufgabe erfolgt durch die Merkmale des Anspruchs 1.

Die erfindungsgemäße Vorrichtung kann mit einer Vielzahl von Geräten, insbesondere mit allen medizinischen Instrumenten verwendet werden, die einen integrierten Arbeitskanal zur Lichtein- und Lichtdurchleitung aufweisen. Hierbei ist die erfindungsgemäße Vorrichtung unabhängig von der konstruktiven und funktionellen Einzelheiten der medizinischen Instrumente, wie insbesondere deren Länge, deren Querschnitt und deren Material anwendbar.

Durch die erfindungsgemäße Vorrichtung wird die Faseroptik am medizinischen Instrument auf einfache und lösbare Art und Weise fixiert und ermöglicht darüber hinaus eine Manipulation des austretenden Lichtes mit dem Instrument, da die Faseroptik mit ihrem distalen Ende fixiert und zentrisch im Arbeitskanal des medizinischen Instrumentes ausgerichtet ist.

Desweiteren ergibt sich der Vorteil, daß die Lichtleitfaser bzw. das Lichtleitfaserbündel der Faseroptik mit Hilfe der erfindungsgemäßen Vorrichtung gas- bzw. flüssigkeitsdicht abgeschlossen ist.

Ferner schützt die erfindungsgemäße Vorrichtung gleichzeitig die Faseroptik vor mechanischen Einflüssen.

Schließlich ergibt sich durch das aufblasbare Ende des Schlauches der erfindungsgemäßen Vorrichtung die Möglichkeit, das freie Lumen eines Arbeitskanales eines Gerätes, insbesondere eines medizinischen Instrumentes, abzudichten.

Insbesondere bei medizinischen Geräten wird mithin der Anwendungsbereich der erfindungsgemäßen Vorrichtung und damit beispielsweise der Anwendungsbereich von Lasern in der medizinischen Endoskopie beträchtlich erweitert. So kann beispielsweise mit Hilfe der erfindungsgemäßen Vorrichtung in dem Arbeitskanal eines Endoskopes ein Laserimpuls über eine Laserfasersonde an einen Krankheitsherd mit der Möglichkeit der Beobachtung übertragen werden.

Die Unteransprüche haben vorteilhafte Weiterbildungen der Erfindung zum Inhalt.

Durch die Aufblasbarkeit des distalen Endes des Schlauches wird es möglich, daß der Außendurchmesser des distalen Endes (das dem Gerät benachbarte Ende) aufgeweitet wird, bis er den Innenquerschnitt beispielsweise eines Arbeitskanales des Gerätes bzw. medizinischen Instrumentes ausfüllt. Damit ist gleichzeitig eine Abdichtung des Arbeitskanales und eine Fixierung der Faseroptik möglich.

Um den Innendruck des Schlauches, der die Faseroptik enthält, erhöhen zu können, kann der Schlauch mit einer Gasdruckquelle verbunden sein.

Hierzu kann entweder ein am proximalen Ende (dem Gerät abgewandten Ende) angebrachter Faserstecker verwendet werden, der einerseits zum Anschluß an die Lichtquelle dient und andererseits mit einem Druckanschluß versehen sein kann.

Als Alternative hierzu ist es jedoch auch möglich, an die Schlauchleitung Enden ein Anschlußteil, beispielsweise in Form eines Druckanschlußverteilers, vorzusehen, der an die Druckquelle angeschlossen werden kann.

In beiden zuvor genannten alternativen Ausführungsformen ist es möglich, die Druckquelle durch entsprechende Steuereinrichtungen anzuschließen und abzutrennen, um gezielt das distale Ende des Schlauches aufblasen zu können bzw. eine Druckminderung vorzunehmen.

Der Schlauch besteht bevorzugterweise aus Kunststoff, kann im Bedarfsfalle jedoch auch aus einem anderen Material bestehen. Die erfindungsgemäße Vorrichtung bietet hierbei einen weiten Rahmen von Anpassungsmöglichkeiten ihrer Komponenten an die jeweiligen Anwendungsfälle.

Schließlich ist es möglich, die erfindungsgemäße Vorrichtung mit ihren Komponenten in Form eines integrierten Bauteiles auszubilden, das beispielsweise die Form einer Laser-Sonde haben kann.

Weitere Einzelheiten, Merkmale und Vorteile der Erfindung ergeben sich aus nachfolgender Beschreibung eines Ausführungsbeispieles anhand der Zeichnung.

Es zeigt:
- Fig. 1: eine schematisch stark vereinfachte Darstellung einer erfindungsgemäßen Vorrichtung, und
- Fig. 2: eine vergrößerte teilweise geschnittene Darstellung des distalen Endes der Vorrichtung.

Die in der Figur dargestellte Ausführungsform einer Vorrichtung 1 ist zur Verbindung einer Faseroptik mit einem Gerät 3 vorgesehen, das insbesondere als medizinisches Instrument, beispielsweise als Endoskop, ausgebildet sein kann.

Die Vorrichtung 1 weist eine Lichtleitfaser bzw. ein Faserbündel 2 auf, das in einem Schlauch 4 angeordnet ist. Der Schlauch 4 kann aus Kunststoff bestehen und weist ein dem Gerät benachbartes distales Ende 5 auf. Ein dem distalen Ende 5 gegenüberliegendes proximales Ende der Vorrichtung 1, bzw. in diesem dargestellten Fall ihrer Lichtleitfaser 2, ist mit 6 bezeichnet.

Das distale Ende 5 der Vorrichtung 1 kann in seinem Querschnitt gegenüber dem Schlauchquerschnitt erweitert werden. Dabei kann das distale Ende 5 eine Form ähnlich einem Ballonkatheter annehmen. Die Querschnittserweiterung des distalen Endes 5 dient dazu, dieses mit dem in das distale Ende 5 hineinlaufenden Ende des Faserbündels 2 in einem Arbeitskanal 7 des Gerätes 3 zu fixieren. Durch die Aufweitung des distalen Endes 5 wird hierbei gleichzeitig der Arbeitskanal 7 des Gerätes 3 abgedichtet.

Durch die beschriebene Festlegung des an seinem Abschluß 8 druckdicht verschlossenen Endes 5 im Arbeitskanal 7 ist ferner eine zentrische Positionierung der Lichtleitfaser 2 erreichbar. Diese wiederum ermöglicht es, die Faseroptik im praktischen Einsatz gezielt bei der Verwendung zu manipulieren, was bei bisher bekannten Anordnungen unmöglich oder sehr schwierig war.

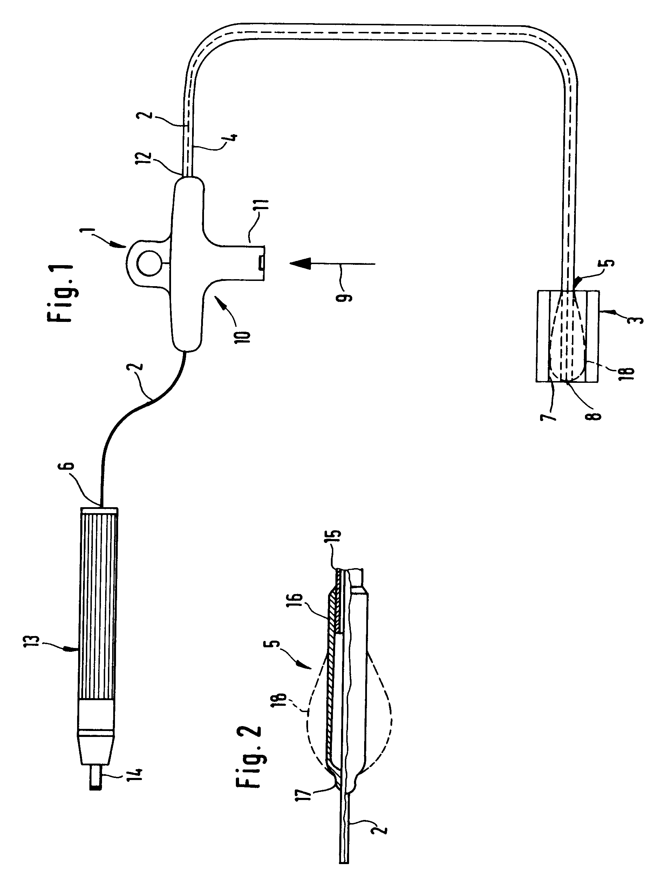

Zum Aufblasen des distalen Endes 5 kann die Vorrichtung 1 mit einer Druckquelle 9 verbunden werden, die in Fig. 1 schematisch durch einen Pfeil angedeutet ist. Bei der dargestellten Ausführungsform ist die Vorrichtung 1 ferner mit einem Druckanschlußverteiler 10 versehen. Dieser ist an ein Ende 12 der bis zum Gerät 3 durchgehenden Schlauchleitung 4 angeschlossen. Es handelt sich hierbei um einen Verteiler, dessen freier Anschluß 11 mit der Druckquelle 9 verbunden werden kann. Der Druckverteileranschluß 10 kann im Einsatz so betrieben werden, daß von der Druckquelle 9 aus eine Innendruckerhöhung des Schlauches 4 erfolgt, die zum Aufblasen des distalen Endes 5 in der zuvor beschriebenen Art und Weise herangezogen werden kann.

Alternativ hierzu ist es auch möglich, einen am proximalen Ende 6 angebrachten Faserstecker 13 mit einem in der Zeichnung nicht näher dargestellten Druckanschluß zu versehen, der dann mit der Druckquelle 9 gesteuert verbunden werden kann. Hierzu ist es möglich, den Faserstecker 13 mit geeigneten Schalt- und Absperrorganeinrichtungen, wie bespielsweise Ventilen, zu versehen, die ein gezieltes Aufblasen des distalen Endes 5 bzw. eine gezielte Druckminderung im Schlauch 4 möglich machen. In diesem Fall verläuft der Schlauch 4 mit seinem Ende 12 bis zum Faserstecker 13.

Der Faserstecker 13 ist ferner mit einem Anschluß 14 versehen, der dazu dient, die gesamte Vorrichtung mit einer Lichtquelle, beispielsweise einem Laser, zu verbinden.

Die erfindungsgemäße Vorrichtung 1 stellt eine universelle Adaptionseinrichtung beispielsweise zwischen einer mit einem Laser verbundenen Faseroptik und einem medizinischen Instrument mit einem Arbeitskanal dar. Hierbei ermöglicht die Vorrichtung 1 eine Fixierung der Faseroptik in dem medizinischen Instrument durch eine druckgesteuerte Formanpassung, die universell einsetzbar ist, da sie unabhängig von der konstruktiven und funktionellen Ausführung des Gerätes bzw. medizinischen Instrumentes ist, mit dem die Faseroptik kombiniert werden soll.

In Fig. 2 ist eine mögliche Ausführung des distalen Endes 5 der erfindungsgemäßen Vorrichtung 1 dargestellt. Die Darstellung der Fig. 2 verdeutlicht das in das Gerät 3 hineinlaufende Schlauchende 15, auf dem ein Endteil 16 unter Bildung einer Dichtung befestigt ist. Hierzu ist beispielsweise eine Klebeverbindung zwischen dem Schlauchende 15 und dem Endteil 16 denkbar. Das Endteil 16 weist eine vordere Spitze 17 auf, die dicht mit der hindurchlaufenden Lichtleitfaser 2 verbunden ist. Dies bedeutet mit anderen Worten, daß durch das Aufbringen des Endteiles 16 auf das Schlauchende 15 und die Befestigung an der Lichtleitfaser 2 im Bereich der Spitze 17 (z.B. durch Aufschrumpfung oder Festklebung) das Schlauchende 15 druckdicht verschlossen ist.

Das kappen- bzw. hülsenartig ausgebildete Endteil 16 besteht aus einem Material, das weicher ist, als das Material des Schlauches 4. Dadurch wird es möglich, den Innendruck im Schlauch 4 über den Druckanschlußverteiler 10 und die Druckquelle 9, beispielsweise in Form einer Spritze, zu erhöhen, ohne daß sich der Schlauch 4 aufbläht. Aufgrund des weicheren Materials des Endteiles 16 jedoch wird dieses bei Innendruckerhöhung im Schlauch 4 aufgeblasen und in seinem Querschnitt erweitert. Daraus ergibt sich die Bildung eines in den Fig. 1 und 2 gestrichelt dargestellten Ballons 18. Dieser ermöglicht die Festlegung und Abdichtung des distalen Endes 5 im Arbeitskanal 7 des Gerätes 3.

## Patentansprüche

1. Vorrichtung (1) zur Verbindung einer eine Lichtleitfaser (2) oder ein Faserbündel (2) enthaltenden Faseroptik mit einem einen Arbeitskanal (7) als optischen Eingang enthaltenden Gerät (3), insbesondere einem medizinischen Instrument, gekennzeichnet durch einen die Lichtleitfaser (2) oder das Faserbündel (2) aufnehmenden Schlauch (4), dessen distales Ende (5) ein kappen. bzw. hülsenartiges Endteil (16) aufweist, das durch Erhöhung des Schlauchinnendruckes aufblasbar und in seinem Durchmesser an den Innendurchmesser des Arbeitskanales (7) anpaßbar ist.

2. Vorrichtung nach Anspruch 1, gekennzeichnet durch eine mit dem Schlauch (4) verbindbare Gasdruckquelle (9).

3. Vorrichtung nach Anspruch 1 oder 2, dadurch gekennzeichnet, daß das proximale Ende (6) der Lichtleitfaser (2) mit einem Faserstecker (13) versehen ist.

4. Vorrichtung nach Anspruch 1 oder 2, dadurch gekennzeichnet, daß der Schlauch (4) über einen Druckanschlußverteiler (10) mit der Druckquelle (9) verbunden ist.

5. Vorrichtung nach einem der Ansprüche 1 bis 4, dadurch gekennzeichnet, daß der Schlauch (4) aus Kunststoff besteht.

6. Vorrichtung nach einem der Ansprüche 1 bis 5, dadurch gekennzeichnet, daß der Schlauch (4), die Faseroptik, der Faserstecker (13) und gegebenenfalls der Druckanschlußverteiler (10) zu einem integralen Teil in Form einer Sonde kombiniert sind.

7. Vorrichtung nach einem der Ansprüche 1 bis 6, dadurch gekennzeichnet, daß das Endteil (16) aus einem Material besteht, das weicher ist als das Material des Schlauches (4).

8. Vorrichtung nach Anspruch 7, dadurch gekennzeichnet, daß das Endteil (16) druckdicht mit dem Schlauchende (15) und dem durchlaufenden Lichtleitfaserbündel (2) verbunden ist.

9. Vorrichtung nach Anspruch 7 oder 8, dadurch gekennzeichnet, daß das Endteil (16) durch Innendruckerhöhung des Schlauches (4) zu einem Ballon (18) aufblasbar ist.

## Claims

1. Apparatus (1) for connecting a fibre optic containing an optical fibre (2) or a fibre bundle (2) to a device (3) containing an operative channel (7) as the optical input, in particular a medical instrument, characterized by a hose (4) which receives the optical fibre (2) or the fibre bundle (2) and whereof the distal end (5) has a cap- or sleeve-like end part (16) which can be inflated by increasing the internal pressure of the hose and can be matched in its diameter to the internal diameter of the operative channel (7).

2. Apparatus according to Claim 1, characterized by a gas pressure source (9) which can be connected to the hose (4).

3. Apparatus according to Claim 1 or 2, characterized in that the proximal end (6) of the optical fibre (2) is provided with a fibre plug (13).

4. Apparatus according to Claim 1 or 2, characterized in that the hose (4) is connected to the pressure source (9) by way of a pressure connection distributor (10).

5. Apparatus according to one of Claims 1 to 4, characterized in that the hose (4) is made of synthetic material.

6. Apparatus according to one of Claims 1 to 5, characterized in that the hose (4), the fibre optic, the fibre plug (13) and where appropriate the pressure connection distributor (10) are combined into an integral part in the form of a probe.

7. Apparatus according to one of Claims 1 to 6, characterized in that the end part (16) is made from a material which is softer than the material of the hose (4).

8. Apparatus according to Claim 7, characterized in that the end part (16) is connected in pressure-tight manner to the hose end (15) and the optical fibre bundle (2) running through.

9. Apparatus according to Claim 7 or 8, characterized in that the end part (16) can be inflated into a balloon (18) by increasing the internal pressure of the hose (4).

## Revendications

1. Dispositif (1) pour connecter un système optique sur fibres comportant une fibre optique (2) ou un faisceau (2) de fibres optiques à un appareil (3), notamment un instrument de médecine, pourvu d'un conduit de travail (7) comme entrée optique, caractérisé par une gaine (4) souple qui reçoit la fibre optique (2) ou le faisceau (2) de fibres optiques et dont l'extrémité distale (5) est munie d'une pièce d'extrémité (16) en forme de coiffe ou de manchon qui peut être gonflée par élévation de la pression à l'intérieur de la gaine et dont le diamètre peut être adapté au diamètre intérieur du conduit de travail (7).

2. Dispositif selon la revendication 1, caractérisé par une source de gaz sous pression (9) qui peut être connectée à la gaine (4) souple.

3. Dispositif selon la revendication 1 ou 2, caractérisé par le fait que l'extrémité (6) proximale de la libre optique (2) est pourvue d'un connecteur (13) de fibre optique.

4. Dispositif selon la revendication 1 ou 2, caractérisé par le fait que la gaine (4) souple est connectée à la source de pression (9) par l'intermédiaire d'un répartiteur (10).

5. Dispositif selon l'une des revendications 1 à 4, caractérisé par le fait que la gaine (4) souple est en matière plastique.

6. Dispositif selon l'une des revendications 1 à 5, caractérisé par le fait que la gaine (4) souple, le système optique sur libres, le connecteur (13) de libre optique et éventuellement le répartiteur de pression (10) sont combinés pour former un ensemble intégré se présentant sous la forme d'une sonde.

7. Dispositif selon l'une des revendications 1 à 6, caractérisé par le fait que la partie terminale (16) est en un matériau plus mou que le matériau de la gaine (4) souple.

8. Dispositif selon la revendication 6, caractérisé par le fait la partie terminale (16) est liée de manière étanche à l'extrémité (15) de la gaine souple et au faisceau (2) de fibres optiques qui traverse celle-ci.

9. Dispositif selon la revendication 7 ou 8, caractérisé par le fait que la partie terminale (16) peut être gonflée pour former un ballon (18) par élévation de la pression intérieure de la gaine souple (4).
